# EUROPÄISCHE PATENTSCHRIFT

(11) **EP 2 571 986 B1**
(45) Veröffentlichungstag und Bekanntmachung des Hinweises auf die Patenterteilung: **30.11.2016**
(21) Anmeldenummer: 11719214.6
(22) Anmeldetag: 26.04.2011
(51) Int. Cl.: C12N 15/10, G01N 33/543, G01N 33/58

(54) **KONTROLLMATERIAL FÜR NUKLEINSÄURETESTE**
Control material for nucleic acid tests
Procédé destiné à la régénération de tissus

(30) Priorität: 20.05.2010 EP 10005296
(43) Veröffentlichungstag der Anmeldung: 27.03.2013
(73) Patentinhaber: Siemens Healthcare Diagnostics Inc., Deerfield, IL 60015-0778 (US)
(72) Erfinder: REIFENBERGER, Elke, 53757 Sankt Augustin (DE); PETRY, Christoph, 41468 Neuss (DE); SCHWERS, Stephan, 50827 Köln (DE); BOHMANN, Kerstin, 50677 Köln (DE)
(74) Vertreter: Schweitzer, Klaus
(86) Internationale Anmeldenummer: PCT/EP2011/056512
(87) Internationale Veröffentlichungsnummer: WO 2011/144416

(56) Entgegenhaltungen:
- WO-A1-2005/054860
- WO-A1-2009/012185
- WO-A2-2006/031815
- US-A1- 2006 199 197
- US-A1- 2009 203 015

### SEQUENCE LISTING

<110> Siemens Healthcare Diagnostics Inc.
<120> Kontrollmaterial für Nukleinsäureteste
<130> 201003087
<160> 2
<170> PatentIn version 3.3
<210> 1
   <211> 360
   <212> DNA
   <213> Homo sapiens
<220>
   <221> regulation element for transcription and RNA stability
   <222> (1) .. (29)
<220>
   <221> regulation element for transcription and RNA stability
   <222> (309)..(360)
<400> 1
   gggagaccac aacggtttcc ctctagaaag gagatcatcg ggacaactct ccttttgatg     60
   gacctggagg aaatcttgct catgcttttc aaccaggccc aggtattgga ggggatgctc    120
   attttgatga agatgaaagg tggaccaaca atttcagaga gtacaactta catcgtgttg    180
   cggctcatga actcggccat tctcttggac tctcccattc tactgatatc ggggctttga    240
   tgtaccctag ctacaccttc agtggtgatg ttcagctagc tcaggatgac attgatggca    300
   tccaagccaa gatctgtacc ccttggggcc tctaaacggg tcttgagggg ttttttgcag    360
<210> 2
   <211> 360
   <212> DNA
   <213> Homo sapiens
<220>
   <221> regulation element for transcription and RNA stability
   <222> (1) .. (29)
<220>
   <221> regulation elements for transcription and RNA stability
   <222> (309)..(360)
<400> 2
   gggagaccac aacggtttcc ctctagaaac ggcacttggg agaagatgtt tgaaaaaact     60
   gactctgcta atgagcctgg actcagagct caagtctgaa ctctacctcc agacagaatg    120
   aagttcatct cgacatctct gcttctcatg ctgctggtca gcagcctctc tccagtccaa    180
   ggtgttctgg aggtctatta cacaagcttg aggtgtagat gtgtccaaga gagctcagtc    240
   tttatcccta gacgcttcat tgatcgaatt caaatcttgc cccgtgggaa tggttgtcca    300
   agaaaagaaa gatctgtacc ccttggggcc tctaaacggg tcttgagggg ttttttggat    360

## Beschreibung

Die vorliegende Erfindung liegt auf dem Gebiet der Molekularbiologie und betrifft ein Kontrollmaterial oder Standardmaterial für Nukleinsäureteste sowie ein Verfahren zu dessen Herstellung.

Molekularbiologische Methoden gewinnen in der klinischen Diagnostik von Erkrankungen zunehmend an Bedeutung. Besonders Verfahren der Nukleinsäureanalytik, wie z. B. Hybridisierungs- oder Amplifikationstechniken wie PCR (Polymerase Chain Reaction), RT-PCR (Reverse Transkription PCR), qPCR (quantitative PCR), TMA (Transcription mediated amplification), LCR (Ligase Chain Reaction), bDNA (branched DNA), Microarrays oder NASBA (Nucleic Acid Sequence Based Amplification) werden zum Beispiel zum Nachweis von Infektionserregern, zum Nachweis von zirkulierenden Tumorzellen, zur Identifizierung von genetischen Risikofaktoren für die Prädisposition einer Erkrankung oder zur Ermittlung sogenannter Genexpressionsprofile angewendet. Die Bestimmung der Genexpression, d. h. die quantitative Bestimmung von Transkripten in Zellen oder Geweben, ermöglicht die Erforschung und Diagnostik von zahlreichen Erkrankungen, wie z. B. von Tumorerkrankungen. Sogenannte Mikroarray- oder Multiplex-Systeme ermöglichen es, in einem einzigen Ansatz die Expression von hunderten oder sogar tausenden von Genen zu bestimmen.

Die Bestimmung der Genexpression oder der Nachweis von Genmutationen in Zellen oder Geweben ist ein komplizierter analytischer Prozess, der üblicherweise mindestens folgende Schritte umfasst: a) Isolierung des Nukleinsäurematerials (RNA oder DNA), b) Prozessierung des Nukleinsäurematerials (z. B. Markierung mit signalgebenden Substanzen, Restriktion, Amplifikation etc.), c) Durchführung einer Nachweisreaktion (Hybridisierung an eine Festphase, Detektion eines Signals) und d) Auswertung der Rohdaten mittels elektronischer Datenverarbeitung.

In der Tumordiagnostik werden häufig in Formalin fixierte und in Paraffin eingebettete Gewebeproben verwendet (formalinfixed paraffin-embedded, FFPE-Proben). Bei der Probenentnahme am Patienten, z. B. bei Biopsieentnahme oder intraoperativer Probenentnahme von Tumormaterial, wird Gewebematerial mit Formalin fixiert und in Paraffin eingebettet, um das Probenmaterial haltbar zu machen. FFPE-Proben sind das Ausgangsmaterial für Paraffin-Schnitte, die als Routineproben in histopathologischen Untersuchungen verwendet werden. Durch die Formalinfixierung kommt es zu einer extremen Kreuzvernetzung von Biomolekülen (Nukleinsäuren mit Proteinen sowie jeweils Proteine oder Nukleinsäuren untereinander). Diese vernetzten Strukturen innerhalb und ausserhalb von Zellen tragen zur Entstehung von unlöslichem bzw. nicht oder schwer lysierbarem Debris bei. Von den im Paraffin eingebetteten Proben werden üblicherweise Schnitte zur Begutachtung durch Pathologen angefertigt. Diese Schnitte können jedoch auch als Ausgangsmaterial in der Nukleinsäureanalytik dienen. Dabei muss bei der Aufreinigung der Nukleinsäuren nach der Lyse sowohl zelluläres Debris als auch das Paraffin entfernt werden. Die Isolierung und Reinigung von Nukleinsäuren aus FFPE-Proben oder -Schnitten ist bekanntermaßen ein kritischer Schritt in der Nukleinsäurediagnostik, da eine mangelhafte Reinigung des Nukleinsäurematerials die nachfolgende quantitative oder qualitative Nachweisreaktion negativ beeinflussen kann.

In analytischen Verfahren wird üblicherweise neben dem zu analysierenden Probenmaterial ein möglichst artgleiches Kontrollmaterial mitgeführt, welches vor dem analytischen Prozess möglichst genauso behandelt wird wie die zu analysierende Probe. Im Falle der Analytik von einer FFPE-Gewebeprobe z. B. eines Tumorpatienten wäre daher eine FFPE-Kontroll-Gewebeprobe das Kontrollmaterial der Wahl. Die Erzeugung von FFPE-Kontroll-Gewebeproben ist jedoch problematisch. Zur Verfügung stehende Gewebeproben sind in der Regel variabel, was ihre Herkunft betrifft. Verschiedene Bereiche eines Tumorgewebes können z. B. unterschiedliche Zellpopulationen bzw. unterschiedliche Zelldichten und damit eine breite quantitative und qualitative Varianz insbesondere im Hinblick auf die Genexpression aufweisen. Zum anderen ist die ausreichende Verfügbarkeit solcher Gewebeproben nicht gewährleistet. Somit ist die Herstellung von größeren Chargen eines Kontroll- oder Standardmaterials mit einer einheitlichen Zusammensetzung basierend auf Gewebeproben nicht möglich.

Es besteht daher ein Bedarf nach einem Kontroll- oder Standardmaterial, das sich für Nukleinsäurebestimmungen, insbesondere für die quantitative Bestimmung von RNA in FFPE-Proben eignet und das reproduzierbar, in größeren Mengen herstellbar ist. Darüber hinaus gibt es auch beim Nachweis bestimmter Mutationen in Tumorproben (beispielsweise von kRAS Mutationen bei kolorektalen Tumoren zu Vorhersage bestimmter Therapien) einen Bedarf an geeigneten Kontroll- oder Standardmaterialien, die alle analytischen Schritte, inklusive der Nukleinsäureisolation, überprüfen.

Verschiedene Kontrollmaterialien für Nukleinsäurebestimmungen sind bekannt.

Ein bekanntes Kontroll- oder Standardmaterial für quantitative RNA-Bestimmungen mittels RT-kPCR oder in einem Mikroarray-System sind in vitro synthetisierte RNA Transkripte. Die Verwendung synthetischer Transkripte hat den Vorteil, das jede gewünschte Sequenz in der gewünschten Quantität bereitgestellt werden kann. Synthetische Oligonukeotide können dazu in geeignete Vektoren (z. B. in pBluescript II KS (+/-), Stratagene) kloniert werden, die anschließend als Ausgangsmaterial für die in vitro-Transkription dienen. Die in vitro-Transkripte müssen dann noch in geeigneter Konzentration in einem geeigneten Puffer verdünnt werden. Nachteilig dabei ist zum einen, dass die RNA sehr instabil ist und wegen der ubiquitär verbreiteten RNAsen in Lösung leicht abgebaut wird. Weiterhin nachteilig ist, dass jedes Transkript, das in der Probe bestimmt werden soll, einzeln hergestellt und mit dem geeigneten Puffer vermischt werden muss. Besonders im Hinblick auf die Multiplex-Systeme oder Multiparameter-Assays, wobei sehr viele Gentranskripte (bis zu mehreren Tausend) in einem einzigen Ansatz nachgewiesen werden, ist alleine die Herstellung und Reinigung der einzelnen Transkripte für die Kontrolle mit einem hohen technischen Aufwand verbunden. Die Verwendung von derartigen Kontrollen basierend auf in vitro-Transkripten bei der Analyse von FFPE-Schnitten ist zudem wenig geeignet, da mit diesen Kontrollen nicht der kritische Schritt der Isolierung der Nukleinsäuren aus FFPE-Proben kontrolliert werden kann.

Ein anderes Kontrollmaterial ist in US 2008/0090243 A1 beschrieben. Dieses Material, das auch als "pseudo-tissue sample" bezeichnet wird, besteht aus Zellen einer oder mehr Zelllinien, die fixiert, aggregiert und anschließend in Paraffin eingebettet werden. Dieses Material ist insofern vorteilhaft, als dass es die Kontrolle der Isolierung der Nukleinsäuren aus FFPE-Proben ermöglicht. Allerdings kann sich das Auffinden einer geeigneten Zelllinie bzw. einer geeigneten Kombination von Zelllinien für ein Nachweisverfahren, bei dem eine Vielzahl bestimmter Gentranskripte nachgewiesen werden soll, schwierig gestalten.

Der vorliegenden Erfindung lag daher die Aufgabe zugrunde eine Zusammensetzung bereit zu stellen, die sich als Kontrollmaterial in einem Verfahren zur Bestimmung von Nukleinsäuren eignet und die insbesondere die Kontrolle des Schrittes der Isolierung von Nukleinsäuren aus eingebetteten Proben ermöglicht.

Die Aufgabe wird dadurch gelöst, dass Zellen mindestens einer isolierten Zelllinie mit mindestens einem stabilisierten Nukleinsäuremolekül vermischt werden und dass die Zell-Nukleinsäuremischung anschließend fixiert und in ein Einbettungsmedium eingebettet wird. Dies hat den Vorteil, dass ein Kontrollmaterial erhalten wird, welches auch als Kontrolle für extrem instabile Nukleinsäuremoleküle, wie z. B. RNA-Moleküle, dienen kann.

Die vorliegende Erfindung betrifft demnach eine Zusammensetzung zur Verwendung als Kontrollmaterial in einem Verfahren zur Bestimmung von Nukleinsäuren enthaltend fixierte Zellen mindestens einer isolierten Zelllinie in einem Einbettungsmedium, die dadurch gekennzeichnet ist, dass ferner gentechnisch oder synthetisch hergestellte und stabilisierte Nukleinsäuremoleküle mindestens einer Spezies in dem Einbettungsmedium enthalten sind, wobei die Nukleinsäuremoleküle durch Komplexierung mit einem kationischen synthetischen Polymer oder mit einem Polyamidoamin-Dendrimer stabilisiert sind.

Der Begriff "isolierte Zelllinie" umfasst genotypisch identische Säugerzellen, insbesondere humane Zellen, die außerhalb des Organismus in einem Nährmedium kultivierbar sind. Der Begriff umfasst ferner Hybrid-Zelllinien, die z. B. durch Fusion von zwei unterschiedlichen Zelltypen herstellbar sind. Bevorzugte Zelllinien sind humane Tumorzelllinien. Die erfindungsgemäße Zusammensetzung enthält fixierte Zellen mindestens einer isolierten Zelllinie. In besonderen Ausführungsformen kann die Zusammensetzung fixierte Zellen einer Mischung von unterschiedlichen isolierten Zelllinien enthalten. Dabei kann es sich um eine Mischung von zwei, drei, vier oder mehr unterschiedlichen Zelllinien handeln.

Der Begriff "Einbettungsmedium" umfasst wasserlösliche und wasserunlösliche Einbettungsmedien. Geeignete wasserlösliche Einbettungsmedien sind z. B. Polyvinylalkohol, Polyethylenglykole, Gelatine und Agar. Geeignete wasserunlösliche Einbettungsmedien sind z. B. Paraffin, Epoxyharze. Die Wahl des Einbettungsmediums hängt davon ab, in welchem Einbettungsmedium die zu untersuchende Patientenprobe eingebettet ist.

In einer bevorzugten Ausführungsform umfasst die erfindungsgemäße Zusammensetzung Paraffin als Einbettungsmedium und wird in Form eines Blocks bzw. Würfels gebracht. Das würfelförmige Material eignet sich z. B. zur Herstellung von Schnitten, die dann als Ausgangsmaterial für die Nukleinsäureisolierung und die weiteren nukleinsäureanalytischen Prozessschritte verwendet werden können.

Die Fixierung der Zellen kann auf verschiedene Art und Weise erfolgen, beispielsweise durch Inkontaktbringen der Zellen mit einem fixierenden Agens. Fixierende Agentien sind z. B. Formaldehyd, Formalin, Glutaraldehyd und Acetaledhyd. Auf welche Art und Weise die Zellen der erfindungsgemäßen Zusammensetzung zu fixieren sind hängt davon ab, wie die zu untersuchende Patientenprobe fixiert ist.

Der Begriff "Nukleinsäuremolekül" umfasst oligomere und polymere Ribonukleotide (RNA), 2'-Desoxyribonukleotide (DNA), Peptid-Nukleotide (PNA) oder Locked-Nukleinsäuren (LNA) einer bestimmten Sequenz mit einer Kettenlänge von mehr als 10 Monomereinheiten. Die Monomereinheiten in RNA oder DNA-Nukleinsäuren sind über Phosphorsäurediesterbindungen zwischen 3'- und 5'-Hydroxylgruppe benachbarter Monomereinheiten verknüpft und an das 1'-Atom der jeweiligen Kohlenhydratkomponente ist glykosidisch eine heterocyclische Base gebunden. Bei einem PNA ist das Zucker-Phosphat-Rückgrat durch ein Pseudopeptid ersetzt. Das Rückgrat besteht dabei z. B. aus Aminoethylglycin-Einheiten, die über neutrale Amid-Bindungen (anstelle der geladenen Phosphodiester-Bindungen einer DNA) miteinander verbunden sind. Bei einem LNA ist das C2- und C4-Atom der Ribose über eine Sauerstoff-Methylen-Brücke verbunden. Dadurch wird die Ribose in der 3'-endo-Konformation fixiert. Nukleinsäuren können durch Ausbildung von intermolekularen Wasserstoffbrückenbindungen Doppel- und Dreifachstränge ausbilden. DNA-Moleküle können beispielsweise synthetisch oder gentechnisch durch Vervielfältigung in einzelligen Wirtsorganismen oder durch in vitro-Amplifikation (PCR) hergestellt werden. RNA-Moleküle können beispielsweise synthetisch oder gentechnisch durch Expression in einzelligen Wirtsorganismen oder durch in vitro-Transkription (PCR) hergestellt werden. PNAs und LNAs werden synthetisch hergestellt.

Der Begriff "Nukleinsäuremoleküle mindestens einer Spezies" umfasst eine Vielzahl von Nukleinsäuremolekülen, d. h. mindestens zwei Nukleinsäuremoleküle derselben Sequenz.

Eine erfindungsgemäße Zubereitung kann mehrere Spezies von Nukleinsäuremolekülen enthalten, z. B. zwei, drei, vier oder mehr Spezies von Nukleinsäuremolekülen mit jeweils unterschiedlicher Sequenz. Im Falle mehrerer Spezies von Nukleinsäuremolekülen kann es sich ausschließlich um DNA-, RNA-, PNA- oder LNA-Moleküle handeln oder um eine beliebige Mischung der verschiedenen Nukleinsäuremolekülarten.

Erfindungsgemäß werden die Nukleinsäuremoleküle stabilisiert, bevor sie mit den Zellen der isolierten Zelllinie vermischt werden. Der Begriff "stabilisiert" umfasst eine Behandlung der Nukleinsäuremoleküle, die dazu führt, dass die Nukleinsäuremoleküle vor einem frühzeitigen Abbau durch z. B. DNAsen, RNAsen oder andere Nukleinsäure-abbauende Enzyme oder durch andere Degradationsprozesse geschützt sind.

Dazu sind die in der Zusammensetzung enthaltenen Nukleinsäuremoleküle durch Komplexierung mit einem kationischen synthetischen Polymer stabilisiert. Geeignete kationische synthetische Polymere sind beispielsweise Polyamin, Polyethylimin, Poly-L-Lysin oder Polyethylenglykol (PEG) -modifiziertes Poly-L-Lysin. Ein besonders bevorzugtes kationisches synthetisches Polymer ist ein Polyamidoamin-Dendrimer.

Eine bevorzugte Ausführungsform der erfindungsgemäßen Zusammensetzung enthält Formalin-fixierte Zellen einer humanen Tumorzelllinie und ein Polyamidoamin-Dendrimerkomplexiertes Transkript (RNA-Molekül) oder Polyamidoamin-Dendrimer-komplexierte DNA eingebettet in Paraffin. Solche Zusammensetzungen eignen sich besonders zur Verwendung als Kontroll- und/oder Standardmaterial in einem nukleinsäurediagnostischen Verfahren zur Charakterisierung von FFPE-Tumorproben.

Ein weiterer Gegenstand der Erfindung ist ein Verfahren zur Herstellung einer erfindungsgemäßen Zusammensetzung. Das Herstellungsverfahren umfasst folgende Schritte:
a) Vermischen einer Suspension von Zellen mindestens einer isolierten Zelllinie mit stabilisierten Nukleinsäuremolekülen mindestens einer Spezies zu einer Zell-Nukleinsäuremolekül-Mischung, wobei die Nukleinsäuremoleküle durch Komplexierung mit einem kationischen synthetischen Polymer oder mit einem Polyamidoamin-Dendrimer stabilisiert sind,
b) Fixierung der Zell-Nukleinsäuremolekül-Mischung,
c) Einbettung der Zell-Nukleinsäuremolekül-Mischung in ein Einbettungsmedium.

In einer bevorzugten Ausführungsform wird die Zell-Nukleinsäuremischung vor der Fixierung zur Aggregation gebracht, indem die Zell-Nukleinsäuremischung mit Blutplasma, bevorzugt mit humanem Blutplasma, vermischt wird und anschließend Thrombin zugegeben wird, wodurch ein Fibringerinnsel entsteht, in welches die Zellen und Nukleinsäuremoleküle eingebettet sind. Dieses Fibringerinnsel, das eine gewebeähnliche Beschaffenheit aufweist, wird dann fixiert und anschließend in ein Einbettungsmedium eingebettet.

In einer weiteren Ausführungsform wird die erfindungsgemäß hergestellte Zusammensetzung in eine würfelförmige Form gebracht (Kantenlänge des Würfels ca. 1 cm).

### FIGURENBESCHREIBUNG

### Figur 1

Diagramm zur Darstellung der Ct-Werte in Abhängigkeit von der Zugabe verschiedener PAMAM-Transkriptmengen zu fixierten MCF-7 Zellen: Die Abbildung zeigt die Roh-Ct-Werte von 5 µm-Schnitten verschiedener FFPE-Blöcke A bis E, die unterschiedliche Mengen (Blöcke A-D) oder gar keine (Block E) zugesetzten PAMAM-komplexierten MMP1- und CXCL13-Transkripte enthalten.

### Figur 2

Diagramm zur Darstellung der Transkriptstabilität in 5 µm-Schnitten eines erfindungsgemäßen FFPE-Blocks enthaltend fixierte Zellen der Brustkrebszelllinie MCF-7 sowie mit einem Polyamidoamin- (PAMAM-) Dendrimer komplexierte MMP1- und CXCL13-Transkripte bei +4 °C.

### BEISPIELE

### Beispiel 1: Herstellung einer erfindungsgemäßen Kontrollzusammensetzung

Zellen der epithelialen Brustkrebszelllinie MCF-7 (etabliert aus einem metastasierten Brustkarzinom in the 1970er Jahren; erhältlich bei der Deutschen Sammlung von Mikroorganismen und Zellkulturen GmbH, Hinterlegungsnummer DSM ACC115) wurden gemäß den Anweisungen der DSMZ kultiviert. Die Zellen wurden geerntet und zweimal mit PBS-Puffer gewaschen. 3×10⁶ Zellen wurden in 400 µl Humanplasma resuspendiert.

2 ng bzw. 20 ng artifizieller 360 nt in vitro-Transkripte mit MMP1 bzw. CXCL13 Sequenzen wurden mit 148 µg PAMAM-G4 Dendrimer (Polyamidoamin Dendrimer der Generation 4, Molekulargewicht 14.215 Dalton, Durchmesser 45 nm, NH₂-Oberflächengruppen: 64; Dentritech Inc., USA) in Tris-Puffer, pH 7,4 vermischt, für eine Stunde bei Raumtemperatur inkubiert und anschließend mit der Zell-Humanplasma-Suspension sorgfältig vermischt. Dieser Mischung wurde humanes Thrombin (40 µl, Sigma, # T4393-100UN) hinzugefügt, und es wurde für 5 Minuten bei 4 °C inkubiert. Das entstandene Fibringerinnsel, wurde als Block aus dem Reaktionsgefäß gelöst und über Nacht in 4 % PBS-gepuffertem Formalin fixiert. Anschließend wurde der Block in einer aufsteigenden Ethanolreihe dehydriert (bis 100 % Ethanol), dann für 2 Stunden in Xylol inkubiert (bis der Block transparent wurde) und schließlich in Paraffin eingebettet. 5-10 µm Schnitte wurden mit einem Standard-Mikrotom erstellt. Das Verfahren zur Einbettung von reinen Zelllinienzellen ist in Koch, I. et al., 2006, Diagn Mol Pathol, 15 (3): 149-156 beschrieben.

MMP1 und CXCL13 wurden ausgewählt, weil es sich dabei um für die Brustkrebsdiagnostik wichtige Transkripte handelt, diese aber nur schwerlich in Brustkrebszelllinien oder anderen Tumorzelllinien zu finden sind. Beispielsweise sind diese beiden Transkripte auch nicht in MCF-7 Zellen nachweisbar.

SEQ ID 1 und SEQ ID 2 zeigen die Sequenzen der artifiziellen MMP1- und CXCL13-Transkripte, die verwendet wurden. Sie bestehen jeweils aus einem Teil der publizierten cDNA-Sequenz der entsprechenden Gene (MMP1: NM002421; CXCL13: NM006419) und darüber hinaus beinhalten sie noch artifizielle Sequenzen, die der Transkript-Stabilität dienen, die aber für die vorliegende Erfindung nicht erforderlich sind.

Zur RNA-Isolierung aus den FFPE-Schnitten wurde die von Bohmann, K. et al. beschriebene automatisierte Methode verwendet (Clin Chem. 2009, 55 (9) :1719-27). Bei dieser Methode handelt es sich um eine vollautomatische Isolierungsmethode für Gesamt-RNA aus FFPE Gewebe, die magnetische Partikel verwendet und mit Hilfe eines Hamilton MICROLAB STARlet Roboters (Hamilton Robotics GmbH, Martinsried, Deutschland) durchgeführt wird. Der Pipettierroboter und die meisten verwendeten Puffer und Chemikalien sind Bestandteil des VERSANT^{®} kPCR Systems von Siemens Healthcare Diagnostics. Eine Zusammenfassung der Methode findet sich auch in Schwers, S. et al., Clin Chem 2009, 55 (11): 1995-2003.

Die Menge der zugesetzten MMP1- und CXCL13-Transkripte in den Eluaten der Isolationsmethode wurde mit Hilfe quantitativer Echtzeit 1-Schritt Reverse Transkriptase-Polymerase-Kettenreaktion (RT-PCR) bestimmt. Dafür wurde der SuperScript^{®} III Platinum^{®} One-Step RT-PCR Kit (Invitrogen) nach Angaben des Herstellers verwendet. Die Experimente wurden auf dem ABI Prism^{®} 7900HT Fast Real-Time PCR System (Applied Biosystems) durchgeführt. Folgendes Temperaturprofil wurde verwendet: 30 min bei 50 °C, 2 min bei 95 °C und 40 Zyklen von 15 sec bei 95 °C und 30 sec bei 60 °C. Neben den zugesetzten komplexierten Transkripten CXCL13 und MMP1 wurden intrinsische Transkripte aus MCF-7 Zellen nachgewiesen. Zum einen ESR1, welches ebenfalls eine Rolle bei der Brustkrebsdiagnostik spielt, und zum zweiten sogenannte Housekeeping Gene RPL37A, OAZ1 und CALM2. Die Transkripte der Housekeeping Gene sind universell und gleichmäßig in Tumorzellen vertreten und dienen bei Genexpressionsuntersuchungen von Tumorproben der Referenzierung auf die vorhandene Menge von Tumormaterial in einem Gewebeschnitt.

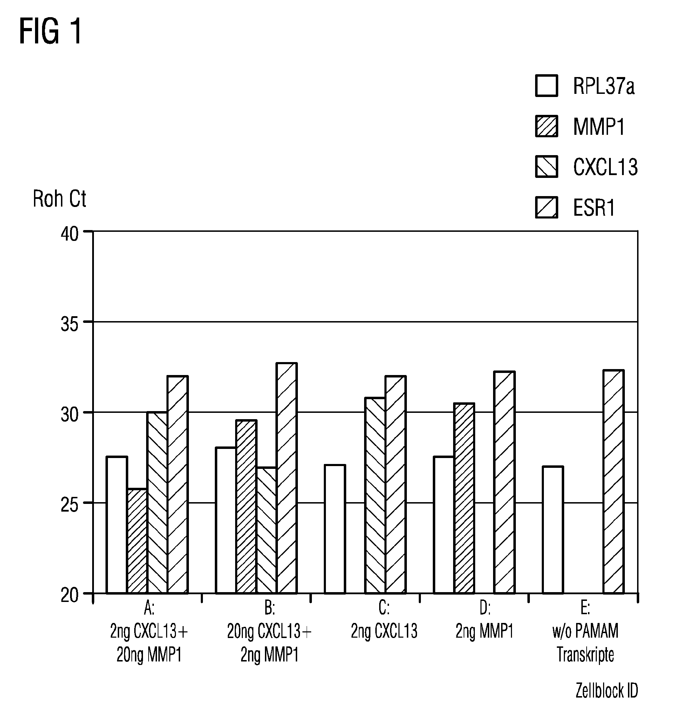

Figur 1 zeigt die Ergebnisse von Schnitten von fünf verschiedenen FFPE-Blöcken, die unterschiedliche Mengen (Blöcke A-D) oder gar keine (Block E) zugesetzten PAMAM-komplexierten MMP1- und/oder CXCL13-Transkripte enthalten. Dargestellt sind Ct-Werte für den Nachweis der Transkripte RPL37A, MMP1, CXCL13 und ESR1. Der cycle treshold (Ct)-Wert (ein Schwellenwert) ist der Teil der quantitativen PCR-Kurve, in dem die Fluoreszenz erstmalig über die Hintergrund-Fluoreszenz ansteigt und sich die Kurve im exponentiellen Bereich befindet. In Schnitten des Blocks E, der keinerlei zugesetzte Transkripte enthält, lassen sich, wie erwartet, nur die Transkripte von RPL37A und ESR1 nachweisen. In Schnitten der Blöcke A-D ist der Ct-Wert, wie erwartet, desto niedriger je mehr Transkript zugesetzt wurde. Eine Verzehnfachung der Menge an Transkript führt zu einer erwarteten Erniedrigung des Ct-Wertes um etwa 3 (theoretisch 3.3). Wie erwartet, kann in den Blöcken, denen nur ein Transkript zugesetzt wurde (C und D), auch nur das eine Transkript nachgewiesen werden.

### Beispiel 2: Stabilität der erfindungsgemäßen Kontrollzusammensetzung

Zur Überprüfung der Stabilität und zur Beantwortung der Frage, ob die zugesetzten PAMAM-komplexierten Transkripte in den Blöcken gleich stabil sind wie die intrinsischen MCF-7-Zelltranskripte wurden 5 µm Schnitte des Blocks A aus Beispiel 1 über einen Zeitraum von 3 Monaten bei 4 °C gelagert. Zu unterschiedlichen Zeitpunkten wurde die RNA aus den FFPE-Schnitten isoliert und eine quantitative Transkriptbestimmung, wie in Beispiel 1 beschrieben, durchgeführt.

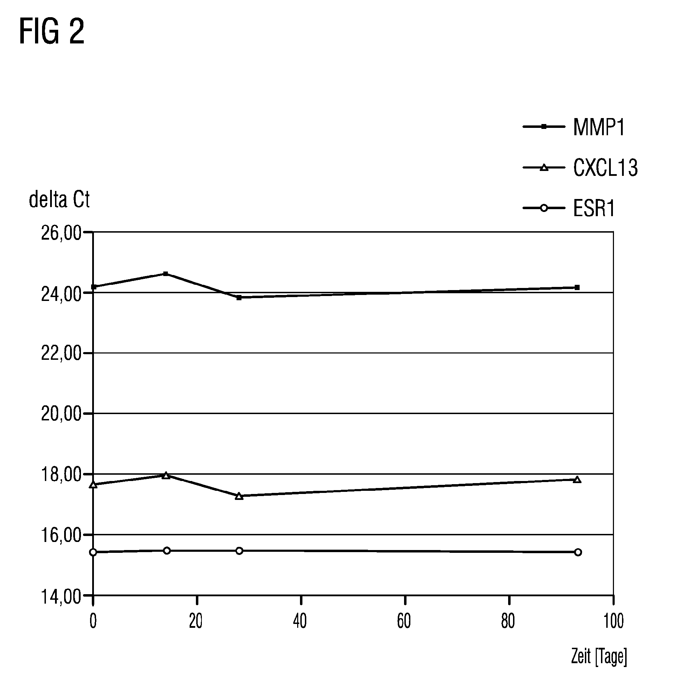

Die Ergebnisse sind in Figur 2 dargestellt. In diesem Fall sind relative (normalisierte) Ct-Werte (delta Ct (=20-[Ct GOI - Mittelwert Ct (RPL37A, CALM2, OAZ1)])dargestellt, um die eventuell variierenden Zellzahlen in den einzelnen Schnitten auszugleichen. Das heißt, die Ct-Werte der brustkrebsrelevanten Transkripte (GOI) wurden ins Verhältnis zu den arithmetischen Mittelwerten der sog. Housekeeping-Transkripte RPL37A, OAZ1 und CALM2 gesetzt.

Wie in Figur 2 zu sehen, verlaufen die Kurven annähernd parallel zur X-Achse, was eine gute Stabilität zeigt. Außerdem unterscheidet sich der Kurvenverlauf für CXCL13 und MMP1 nicht von dem Kurvenverlauf für ESR1. Das heißt, über einen Zeitraum von 3 Monaten zeigte sich kein Stabilitätsunterschied zwischen den zugesetzten PAMAM-komplexierten Transkripten und dem intrinsischen ESR1-Transkript.

## Patentansprüche

1. Zusammensetzung zur Verwendung als Kontrollmaterial in einem Verfahren zur Bestimmung von Nukleinsäuren enthaltend fixierte Zellen mindestens einer isolierten Zelllinie in einem Einbettungsmedium **dadurch gekennzeichnet, dass** ferner gentechnisch oder synthetisch hergestellte und stabilisierte Nukleinsäuremoleküle mindestens einer Spezies in dem Einbettungsmedium enthalten sind, wobei die Nukleinsäuremoleküle durch Komplexierung mit einem kationischen synthetischen Polymer oder mit einem Polyamidoamin-Dendrimer stabilisiert sind.

2. Zusammensetzung nach Anspruch 1 **dadurch gekennzeichnet, dass** das enthaltene Nukleinsäuremolekül durch Komplexierung mit einem kationischen synthetischen Polymer aus der Gruppe Polyamin, Polyethylimin, Poly-L-Lysin und Polyethylenglykol-modifiziertes Poly-L-Lysin stabilisiert ist.

3. Zusammensetzung nach einem der vorhergehenden Ansprüche, wobei die Nukleinsäuremoleküle Moleküle aus der Gruppe RNA, DNA, PNA und LNA sind.

4. Verfahren zur Herstellung einer Zusammensetzung gemäß einem der vorhergehenden Ansprüche, welches folgende Schritte umfasst:
a)Vermischen einer Suspension von Zellen mindestens einer isolierten Zelllinie mit gentechnisch oder synthetisch hergestellten und stabilisierten Nukleinsäuremolekülen mindestens einer Spezies zu einer Zell-Nukleinsäuremolekül-Mischung, wobei die Nukleinsäuremoleküle durch Komplexierung mit einem kationischen synthetischen Polymer oder mit einem Polyamidoamin-Dendrimer stabilisiert sind,
b) Fixierung der Zell- Nukleinsäuremolekül-Mischung,
c) Einbettung der Zell- Nukleinsäuremolekül-Mischung in ein Einbettungsmedium.

5. Verfahren nach Anspruch 4, wobei die Zell-Nukleinsäuremolekül-Mischung vor der Fixierung in Humanplasma suspendiert und durch Zugabe von Thrombin koaguliert wird.

6. Verfahren nach einem der Ansprüche 4 oder 5, wobei die Fixierung durch Inkubation der Zell-Nukleinsäuremolekül-Mischung in Formalin erfolgt.

7. Verfahren nach einem der Ansprüche 4 bis 6, wobei die Einbettung in Paraffin erfolgt.

## Claims

1. A composition for use as control material in a method for determining nucleic acids containing fixed cells of at least one isolated cell line in an embedding medium, wherein genetically engineered or synthetically produced and stabilized nucleic acid molecules of at least one species are additionally present in the embedding medium, the nucleic acid molecules having been stabilized by complexing with a cationic synthetic polymer or with a polyamidoamine dendrimer.

2. The composition as claimed in claim 1, wherein the nucleic acid molecule present has been stabilized by complexing with a cationic synthetic polymer from the group consisting of polyamine, polyethylenimine, poly-L-lysine and polyethylene glycol-modified poly-L-lysine.

3. The composition as claimed in either of the preceding claims, wherein the nucleic acid molecules are molecules from the group consisting of RNA, DNA, PNA and LNA.

4. A method for preparing a composition as claimed in any of the preceding ..claims., comprising the following steps:
a) mixing a suspension of cells of at least one isolated cell line with genetically engineered or synthetically produced and stabilized nucleic acid molecules of at least one species to give a cell/nucleic acid molecule mixture, the nucleic acid molecules having been stabilized by complexing with a cationic synthetic polymer or with a polyamidoamine dendrimer,
b) fixing the cell/nucleic acid molecule mixture,
c) embedding the cell/nucleic acid molecule mixture in an embedding medium.

5. The method as claimed in claim 4, wherein the cell/nucleic acid molecule mixture is suspended in human plasma and coagulated by addition of thrombin prior to the fixation.

6. The method as claimed in either of claims 4 and 5, wherein the fixation is achieved by incubation of the cell/nucleic acid molecule mixture in formalin.

7. The method as claimed in any of claims 4 to 6, wherein the embedding is achieved in paraffin.

## Revendications

1. Composition pour être utilisée comme matériau de contrôle dans un procédé pour déterminer des acides nucléiques contenant des cellules fixées d'au moins une lignée cellulaire isolée dans un milieu d'inclusion, **caractérisée en ce que** des molécules d'acide nucléique stabilisées et fabriquées en outre par génie génétique ou par synthèse d'au moins une espèce sont contenues dans le milieu d'inclusion, dans laquelle les molécules d'acide nucléique sont stabilisées par complexion avec un polymère synthétique cationique ou avec un dendrimère de polyamidoamine.

2. Composition selon la revendication 1, **caractérisée en ce que** la molécule d'acide nucléique contenue est stabilisée par complexion avec un polymère synthétique cationique parmi le groupe de la polyamine, de la polyéthylène-imine, de la poly-L-lysine et de la poly-L-lysine modifiée par polyéthylène-glycol.

3. Composition selon l'une quelconque des revendications précédentes, dans laquelle les molécules d'acide nucléique sont parmi le groupe de l'ARN, l'ADN, le PNA et le LNA.

4. Procédé pour fabriquer une composition selon l'une quelconque des revendications précédentes, lequel comprend des étapes suivantes :
a) le mélange d'une suspension de cellule d'au moins une lignée cellulaire isolée avec des molécules d'acide nucléique stabilisées et fabriquées par génie génétique ou par synthèse d'au moins une espèce pour former un mélange de molécules d'acide nucléique cellulaire, dans lequel les molécules d'acide nucléique sont stabilisées par complexion avec un polymère synthétique cationique ou un dendrimère de polyamidoamine,
b) la fixation du mélange de molécules d'acide nucléique cellulaire,
c) l'inclusion du mélange de molécules d'acide nucléique cellulaire dans un milieu d'inclusion.

5. Procédé selon la revendication 4, dans lequel le mélange de molécules d'acide nucléique cellulaire est mis en suspension avant la fixation dans du plasma humain et est coagulé par ajout de thrombine.

6. Procédé selon l'une quelconque des revendications 4 ou 5, dans lequel la fixation est effectuée par incubation du mélange de molécules d'acide nucléique cellulaire dans de la formaline.

7. Procédé selon l'une quelconque des revendications 4 à 6, dans lequel l'inclusion est effectuée dans de la paraffine.
